# EUROPEAN PATENT APPLICATION

(11) **EP 4 789 601 A1**
(43) Date of publication of application: **12.08.2026**
(21) Application number: 24873929.4
(22) Date of filing: 25.09.2024
(51) Int. Cl.: A61B 1/313, A61B 1/04, A61B 5/00, A61B 90/00

(54) **SYSTEM AND METHOD FOR IDENTIFYING, MEASURING, AND ASSESSING THE THERAPEUTIC RESPONSE OF THE EXTENT AND DISTRIBUTION OF TUMOUR BURDEN IN THE PERITONEAL CAVITY**

(30) Priority: 02.10.2023 BR 102023020323
(71) Applicant: Seitenfus, Rafael, 90420-010 Porto Alegre (BR); De Barros, Eduardo, 91900-200 Porto Alegre (BR); Rehm, Rafael, 90460-110 Porto Alegre (BR)
(72) Inventor: SEITENFUS, Rafael, 90420-010 Porto Alegre (BR); DE BARROS, Eduardo, 91900-200 Porto Alegre (BR); REHM, Rafael, 90460-110 Porto Alegre (BR); FERREIRA, Paulo, 93040-000 São Leopoldo (BR); MACHADO, João, 94465-200 Viamão (BR)
(74) Representative: Pereira Garcia, João Luís
(86) International application number: PCT/BR2024/050441
(87) International publication number: WO 2025/073024

(57) **Abstract**

A computer-implemented system and a method for identifying, measuring, and evaluating the distribution and extent of diseases in peritoneal cavities are described. The method allows obtaining images of the peritoneal cavity (100), previously demarcated by markers (10) fixed by a videolaparoscopic procedure, in which, through computer processing, it is possible to verify the behavior of the peritoneal disease at different moments of the therapy and compare it with a pattern of groups of pathologies stored in a remote database (40), in the cloud, such as a data ecosystem.

## Description

### FIELD OF THE INVENTION.

The present patent of invention describes a system and a method implemented on a computer to identify, measure and evaluate the distribution and extent of diseases in peritoneal cavities. The method allows obtaining images of the peritoneal cavity, previously demarcated by markers fixed by a videolaparoscopic procedure, in which, through computer processing, it is possible to verify the behavior of the peritoneal disease at different moments of the therapy and compare it with a pattern of groups of pathologies stored in a remote database, in the cloud, such as a data ecosystem.

### BACKGROUND OF THE INVENTION.

Tumors in the peritoneal space have their extension and positioning verified by means of imaging tests or by open or minimally invasive surgical techniques with direct or indirect visualization, such as videolaparoscopy, open surgery, computed tomography or magnetic resonance imaging.

Adequate evaluation of the behavior of diseases in the peritoneal space, especially neoplasms, has become a fundamental part of the decision-making process regarding the treatment of pathologies. The adequate evaluation of the degree of involvement of these spaces by pathologies of different etiologies is an additional challenge for measuring the extent of the disease in the space under analysis. In addition, the monitoring of the progression or regression of the involvement of the space under analysis uses subjective and imprecise methods, making it difficult to accurately measure both the progression of the disease and the response to different therapies, such as systemic or intracavitary chemotherapy.

In the case of peritoneal carcinomatosis, this is a disease associated with the spread of cancer cells in the peritoneal space. It is frequently related to tumors originating from the digestive tract (colon, stomach, pancreas and appendix), the gynecological tract (ovaries and fallopian tubes) and primary peritoneal tumors. Peritoneal dissemination of metastases is often classified as stage 4 neoplasia. Thus, the natural history of the evolution of this form of peritoneal dissemination leads to outcomes such as intestinal obstruction, ascites and death. Any neoplasia that allows the proliferation of neoplastic cells in the peritoneal space will develop this form of neoplastic dissemination.

Exact estimates of the index of peritoneal involvement worldwide are scarce in the literature. The data obtained so far allow us to verify that between 15% and 20% of all colon neoplasms develop carcinomatosis, regardless of the treatment method. Furthermore, between 50% and 60% of stomach neoplasms show peritoneal dissemination at diagnosis, and the peritoneal route is the main disseminating route in ovarian neoplasms.

Currently, the only treatment with curative intent for patients with peritoneal carcinomatosis is cytoreductive surgery and hyperthermic intraperitoneal chemotherapy. However, only 10% of all patients with peritoneal carcinomatosis qualify for these procedures. With the incorporation of new technologies and approaches to peritoneal carcinomatosis, both for the adequate selection of patients and for monitoring their response to the proposed therapies, greater precision has been demanded for the evaluation of peritoneal metastases.

The need for precision in measuring both the extent, dissemination and response of peritoneal metastases has brought increasing challenges to current analog tools, which present limitations in their responses. For example, indices are used to analyze peritoneal carcinomatosis, such as Peritoneal Cancer Index (PCI), Fagotti Index and Peritoneal Regression Grading Score (PRGS). However, such indices are subjective and analogical, not allowing the precise measurement and calculation of peritoneal metastases in terms of their number, density, volume and behavior throughout the natural history of neoplasms of different etiologies.

To analyze the response of solid tumors, analog tools such as Recist (Response Evaluation Criteria in Solid Tumors) are provided. This tool has helped to interpret the role of new treatments, being the reference for determining tumor response and the parameter used for phase II and phase III studies. However, Recist has limitations in measuring tumors smaller than 10 mm, cystic lesions and areas with locoregional treatment (D. Jayne. (2007). Molecular Biology of Peritoneal Carcinomatosis. In: Peritoneal Carcinomatosis: Principles of Management, Springer, ch. 2, pp. 1-10. [Online]. https://doi.org/10.1007/978-0-387-48993-3_2), (S. Käbler and J. Jhne.

(2007). Staging and Scoring of Peritoneal Carcinomatosis. In: Peritoneal Carcinomatosis: Principles of Management. Springer, ch. 14, pp. 1-10. [Online]. https://doi.org/10.1007/978-0-387-48993-3_14), (S. Samel and M. Löhr. (2007). Targeted Intraabdominal Chemotherapy for Peritoneal Carcinomatosis. In: Peritoneal Carcinomatosis: Principles of Management, Springer, ch. 32, pp. 1-10. https://doi.org/10.1007/978-0-387-48993-3_32.). All these characteristics determine that peritoneal carcinomatosis is a manifestation considered unmeasurable by Recist, which makes it difficult to apply the tool to evaluate peritoneal carcinomatosis treatments.

In an attempt to quantify carcinomatosis, several scoring systems have been developed (Fujimoto S, Takahashi M, Mutou T, et al. Improved mortality rate of gastric carcinoma patients with peritoneal carcinomatosis treated with intraperitoneal hyperthermic chemoperfusion combined with surgery. Cancer. 1997; 79: 884- 891; Gilly FN, Carry PY, Sayag AC, et al. Regional chemotherapy (with mitomycin C) and intra-operative hyperthermia for digestive cancers with peritoneal carcinomatosis. Hepatogastroenterology. 1994; 41: 124129; Jacquet P, Sugarbaker PH. Clinical research methodologies in diagnosis and staging of patients with peritoneal carcinomatosis. In: PH Sugarbaker, ed. Peritoneal Carcinomatosis: Principles of Management. Boston, MA: Kluwer Academic Publishers; 1996: 359- 374; van der Vange N, van Goethem AR, Zoetmulder FA, et al. Extensive cytoreductive surgery combined with intra-operative intraperitoneal perfusion with cisplatin under hyperthermic conditions (OVHIPEC) in patients with recurrent ovarian cancer: a feasibility pilot. Eur J Surg Oncol. 2000; 26: 663- 668; Goere D, Souadka A, Faron M, et al. Extent of colorectal peritoneal carcinomatosis: attempt to define a threshold above which HIPEC does not offer survival benefit: a comparative study [published online ahead of print January 29, 2015]. Ann Surg Oncol; Verwaal VJ, van Tinteren H, van Ruth S, Zoetmulder FA. Predicting the survival of patients with peritoneal carcinomatosis of colorectal origin treated by aggressive cytoreduction and hyperthermic intraperitoneal chemotherapy. Br J Surg. 2004; 91: 739- 746.). Basically, the systems of quantification developed involve dividing the abdomen into regions. One of the most widely used scoring systems is the Peritoneal Cancer Index (PCI), which divides the peritoneal cavity and small intestine into 13 different regions. Each region is then given a subjective/scalar score from 0 to 3 based on the quantity of tumor present (0 indicates none, while 3 indicates confluent tumor or tumor measuring more than 5!cm).

Although still somewhat subjective and analog, these systems allow surgical oncologists to better compare outcomes, including oncologic outcomes, morbidity, and mortality, based on the extent of tumor burden. PCI is currently the most widely used tool for evaluating carcinomatosis, demonstrating its prognostic value in carcinomatosis of different etiologies. This fact corroborates the need to identify, with better precision, the distribution and volume of the tumor in the peritoneal space.

The state of the art describes methods for diagnosing peritoneal carcinomatosis. Document UA99353 describes a method of radiographic diagnosis of the severity of peritoneal carcinomatosis in colorectal cancer, verifying the amount of free fluid in the abdominal cavity in the presence of nodular lesions of 0.5-1.5!cm in the peritoneum.

Document KR20210039078 describes a peptide probe for the diagnosis of peritoneal carcinomatosis that specifically binds to a gastric cancer cell line and can be used as an imaging index in clinical and endoscopic analyses to diagnose abdominal metastases of gastric cancer.

Document RU2625898 describes a method of scoring the probability of peritoneal carcinomatosis in patients with portal cholangiocarcinoma based on criteria such as disease stage TM (I-IV), metastatic lesion of regional lymph nodes, presence of distant metastatic lesions, lesion of the trunk and branches of the portal vein, presence of ascites.

The state of the art describes subjective methods for describing the physical and volumetric characteristics of peritoneal carcinomatosis. This subjectivity creates difficulty to identify the behavior of pathologies, such as measuring and calculating the size of the incidence, the volume of the nodules and the distribution in a statistical way. Obtaining approximate measurements of the size of the metastases and the large gradient (difference of measurements in each of the quantities analyzed) makes it difficult to accurately measure the tumor burden present in the peritoneal cavity. This imprecision and inference in measurement leads to imprecision in the analysis of the nodule response to treatments.

Recent medical approaches have taken into account individual genetic variation, the environment and lifestyle of each person. This precision medicine, or personalized medicine, allows physicians to more accurately predict which treatments and disease prevention strategies will be effective for a specific patient. In this recent trend, physicians use diagnostic information to better understand a patient's disease, possibly including genetic testing to determine a patient's predisposition to certain diseases or conditions, if it involves the use of targeted treatments, which are designed to attack diseased cells more effectively and with fewer side effects than traditional treatments.

In the context of information technologies and their digital tools and the growth of artificial intelligence (AI), the association of correlated data can be an important means of improving the diagnosis and treatment of cancer, aiding in diagnoses, eliminating or reducing subjectivity, increasing mathematical and statistical precision over time and identifying patterns in the various patients affected by these pathologies. The large volume of data and the possibility of analyzing such data with intelligent algorithms allows expanding the analysis of individual characteristics. The evidence observed in the data set provides information that has an impact for individual intervention, potentially modifying the natural history of the individual's pathology. This phenomenon is observed in the analysis of medical images to detect early signs of breast cancer (Subasi, A., Kandpal, A. D., Raj, K. A., & Bagci, U. (2023). Breast cancer detection from mammograms using artificial intelligence. Elsevier. DOI:10.1016/b978-0-443-18450-5.00005-0.), to detect pulmonary nodules (Zheng, S., van Ooijen, P. M. A., & Oudkerk, M. (2022). Lung Cancer Screening and Nodule Detection: The Role of Artificial Intelligence. Springer International Publishing. DOI:10.1007/978-3-030-92087-6_43) and to detect ovarian cancer. Thus, Artificial Intelligence, through methods implemented in computer algorithms, is consolidating itself as a safe digital technology for diagnosing and monitoring the evolution of tumors.

However, a critical step in the detection and diagnosis of carcinomas concerns the precise identification of the region and characteristics of the metastatic nodules present in the area of interest. Based on current techniques, some patterns related to shape, size, density, extent and spectral evaluation of color, which may allow inferring the benign or malignant nature, are not apparent at first glance, impacting the treatment and therapeutic outcome.

Therefore, the present invention describes a system that includes markers implanted in the peritoneal cavity, a unit for acquisition and pre-processing of images obtained from the body cavity and a post-processing unit with a computer program that analyzes the received data and compares it with reference parameters stored in a database installed on a cloud server, fed by registered users.

The computer-implemented method comprises obtaining digital images of the peritoneal cavity, previously demarcated by the markers implanted through a laparoscopic procedure. The markers are references for determining position and size, allowing the acquisition of precise information on the peritoneal cavity, through digital analysis of the images, followed by image processing and comparison with data stored on a cloud server, in order to establish a set of information and therapies for individual intervention.

### BRIEF SUMMARY OF THE INVENTION.

The present invention describes a system and method for measuring the extent of peritoneal cavity involvement, over time and throughout the application of specific treatments, through the stages of marking regions of interest to define a reference for obtaining images, pre-processing, analysis, processing and comparison with data from a database with relevant information from other patients to identify patterns of evolution of the pathology or otherwise.

The invention describes a system whose data is stored encrypted in a remote database, such as a data ecosystem, which associates information on the natural history of the disease and the treatment of each individual.

The invention describes a computer-implemented method to identify and measure peritoneal cavity involvement using digital mask dissociation and image overlapping to identify geometric, physical and behavioral characteristics, across time, of different pathologies.

The invention describes a system and method that allows associating images and information from populations with similar characteristics for a detailed and accurate description of physical, geometric, distribution and behavior characteristics of the pathology under analysis, in order to document and identify individual or collective characteristics of the pathologies.

The invention describes a minimally invasive method of marking that allows the accurate identification of the region and characteristics of the metastatic nodules present in the area of interest, allowing the precise acquisition of the physical and geometric characteristics and behavior throughout the therapeutic process, based on the identification of patterns that may not be apparent at first glance.

The invention describes a method that allows obtaining data about the total number of nodules in the area of interest, which may indicate a more complex and disseminated condition or a more localized condition, from data related to the volume and percentage of nodules in certain diameter ranges.

The invention describes a method that allows comparing the physical and geometric characteristics of the detected nodules and the changes in response to established treatments, measuring and quantifying the magnitude of the observed response, in order to provide comparative reports of the individual and collective analysis, based on the data stored in an ecosystem.

The invention describes a method that can indicate the severity or extent and the nature (benign or malignant) based on the spectral evaluation of the color and total area of nodules.

### BRIEF DESCRIPTION OF THE FIGURES.

Figure 1 presents a schematic representation of the system, highlighting the basic components defined as marker (10), image acquisition unit (20), pre-processing unit (30) and remote processing unit (40).
Figure 2A shows details of a marker, Figure 2B shows details of the top surface of a marker and Figure 2C shows an example of an arrangement of the positioning of the markers in the peritoneal cavity.

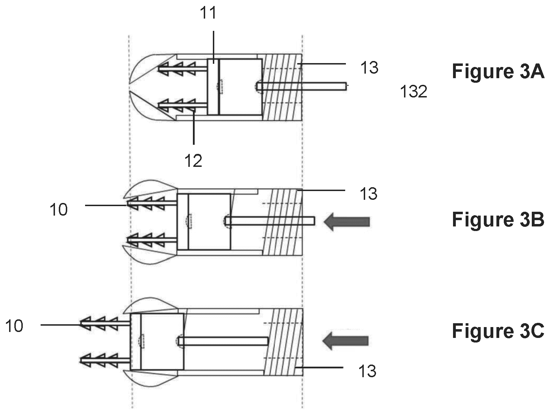

Figure 3A shows the representation of a marker placed in the cartridge, Figure 3B shows the marker being moved from the internal region of the cartridge by exerting manual pressure on the rod and Figure 3C shows the marker in position for application in the peritoneal cavity.

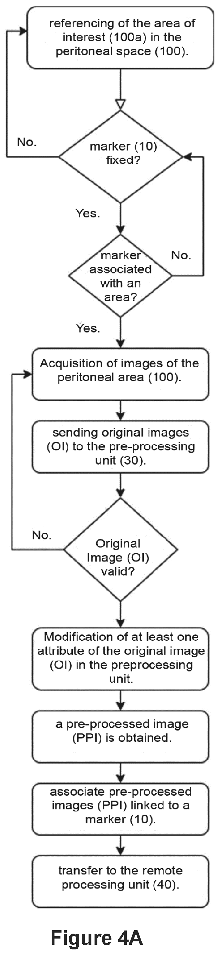

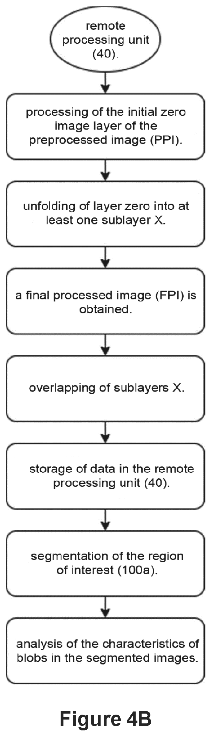

Figure 4A shows a flowchart of the method implemented on a computer to identify, measure and evaluate the therapeutic response of the extent and distribution of the tumor burden in the peritoneal space, highlighting the steps of application of the marker in the peritoneal space to the sending of the pre-processed images (PPI) to the remote processing unit and Figure 4B shows the steps of the method executed in the remote processing unit.
Figure 5A shows the image obtained after validation of the pre-processing system; Figure 5B shows the definition of circular regions on the image obtained by plotting approximately circular regions obtained after calculating the Gaussian difference between the different pixels of the images of layer zero and layer X; Figure 5C shows the thresholding technique in the processed image to identify regions of interest characterized by a certain homogeneity of color intensity (Group of Pixels with Oncological Risk - GPOR).

### DETAILED DESCRIPTION OF THE INVENTION.

For the purposes of the present invention, the following terms are defined:

"Area of interest (100a)" comprises an extension of an individual's peritoneal cavity, demarcated by at least one marker (10), where visual exploration will be performed by acquiring digital images;

Original image (OI) comprises the digital image acquired from the area of interest together with the marker;

Peritoneal Digital Localization (PDL) comprises the position of the areas and elements of interest in relation to the marker (10) in the acquired images.

Group of pixels with oncological risk (GPOR) comprises an area with a certain homogeneity of color intensity of the pixels that correspond to peritoneal nodules or risk of active neoplasia.

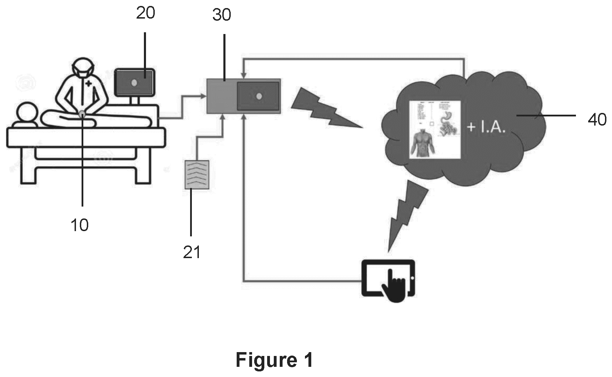

The system for identifying, measuring and evaluating the therapeutic response of the extent and distribution of the tumor burden in the peritoneal space, which is the object of the present invention, comprises at least one marker (10) implanted in the peritoneal cavity (100) of an individual. The marker (10) constitutes a reference in the area of interest (100a) of the peritoneal cavity (100) for acquiring digital images. The digital images obtained by an image acquisition unit (20) and referenced by the marker(s) (10) are sent to a pre-processing unit (30) and then to a remote unit installed in the cloud (40), such as a data ecosystem. Figure 1 shows the schematic representation of the system that is the object of the present invention.

Marker (10) comprises a physical structure with dual function. In a first function, marker (10) demarcates an area of interest (100a) in the peritoneal cavity (100), constituting a reference for establishing the parameters obtained through the method implemented on the computer. In a second function, marker (10) has visual elements that, when analyzed by a computer program, allow validating the acquired image (in the context of the present invention called "original image (OI)") in order for it to be processed in subsequent steps of the method.

Marker (10) has a top surface (11), preferably, but not limited to, a circular surface, and an anchoring means (12) associated with the top surface (11).

Marker (10) is implanted in the peritoneal cavity (100) by a videolaparoscopy procedure. The anatomical location of the marker (10) in the peritoneal space (100) is defined by the user, based on the areas of interest (100a) wished to be explored.

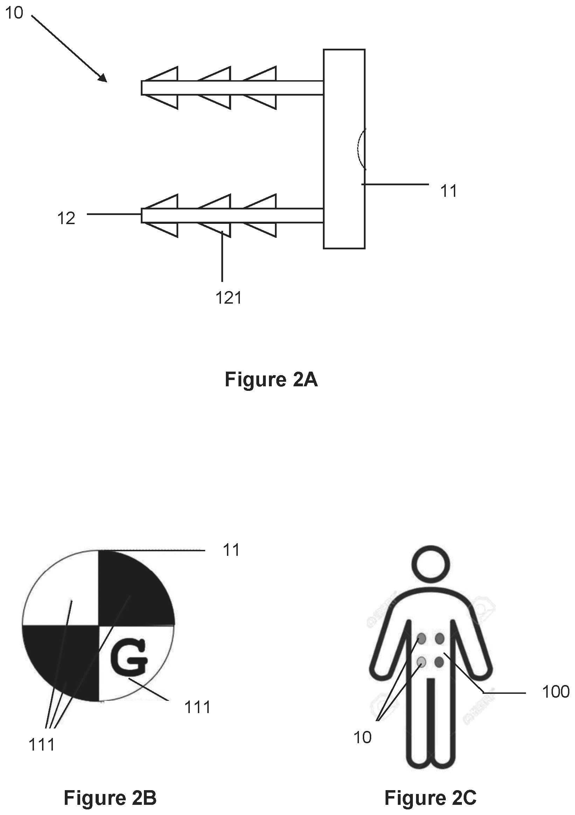

Preferably, the anchoring element (12) of marker (10) has indentations (121), as shown in Figure 2A, ensuring the fixation of marker (10) on the peritoneal wall and the stabilization of the top surface (11).

Stabilization of the top surface (11) of marker (10) is particularly relevant as it references the digital images obtained from the area of interest (100a) in the peritoneal cavity (100) and validates the digital image in the pre-processing unit (30).

Visual elements (111) such as color printing, pictograms or the like are arranged on the top surface (11) of marker (10). The visual elements (111) are recognized in the steps of pre-processing and processing of the digital images to determine their peritoneal location and azimuth. Azimuth is the centering element of the Original Image (OI) for comparison with future images of the same region and the element used to determine the Digital Peritoneal Location (DPL) of the elements of interest in the acquired images. Determination of the intraperitoneal anatomical position of the marker (10) for sectorization, focus, sharpness, angle and ideal distance for capturing the image, reference for Digital Peritoneal Localization (DPL) and reference for calculating the area and volume and determining the group of pixels with oncological risk (GPOR) in the region of interest (100a).

Preferably, the top surface (11) is divided into four quadrants, as shown in Figure 2B.

Preferably, marker (10) is positioned in a cartridge (13) equipped with a plunger (131) which, when actuated by a drive rod (132), moves the marker (10) along the axial plane so that the anchoring element (12) is fixed to the peritoneal wall (100).

An image acquisition unit (20) captures images of the area of interest (100a) of a peritoneal space (100) based on the referencing of at least one marker (10).

The image acquisition unit (20) preferably comprises a videolaparoscopy tower and a rigid scope or rigid endoscope.

An electronic pre-processing unit (30) includes a computing board that executes a computer program that converts the original image (OI) received from the image acquisition unit (20) into a processing format required by the operating system, validates the original image (OI) and modifies one or more attributes of said original image (OI), obtaining a pre-processed image (PI) in accordance with, but not necessarily corresponding to, the Digital Imaging and Communications in Medicine (DICOM) system.

The DICOM system represents a set of standards created to standardize the electronic format used in the storage and communication of images. With standardization, all types of exams - CT scans, MRIs, X-rays, etc. - are stored in a single format, allowing exchange between equipment from different brands. The creation of this set of standards allows the images to be recognized and viewed on any device.

In the context of the present invention, the term "validate" comprises comparing the captured image of the marker (10), which becomes the reference for positioning and image quality, with a standard image stored in the electronic pre-processing unit (30). The ideal position for capturing, identified from each marker (10), becomes the reference for image acquisition, referencing the positioning for the overlapping of future or previous images.

The peritoneal anatomical position of marker (10) is loaded into the pre-processing unit (30) and sent to and stored in the data processing unit (40). During a new application for a given patient, the pre-processing unit (30) retrieves the record of the peritoneal anatomical position of marker (10) stored in the data processing unit (40). In this way, each marker has an identifier, captured in digital images, which allows the reproducibility of its positioning in future events, ensuring the acquisition of a set of images to assess the evolution of the pathology.

In the pre-processing unit (20), an interface is provided for viewing the images captured by the acquisition unit (20) and entering data relating to the patient and the pathology.

For capturing the image by the image pre-processing unit (20), a physical means (21) is provided, such as a button or pedal, to trigger the acquisition of images. Optionally, image capture is performed semiautomatically, with the aid of positional masks that are generated on the interface of the pre-processing unit (30), said masks constituting templates that allow the image acquisition unit (20) to be centered based on the visual references present on the top surface (11) of marker (10).

The human-machine interface enables loading the data related to the identification of the individual undergoing treatment and data related to the treatment and the organization of the image files to be transferred to the remote processing unit (40).

Among the attributes of the original image (OI) that can be changed in the pre-processing unit (30), it is possible to highlight adjustment of the azimuth, focus, sharpness and centering of the image on marker (10).

If the pre-processing unit (30) identifies a non-conformity in relation to the acquired image of marker (10), a signal is sent to the pre-processing unit interface to acquire new images of the area of interest (100a).

The set of pre-processed images (PI) of an area of interest (100a) is stored in an identified file, relating the image and the associated marker (10). The metadata accompanying each image are stored for later retrieval and analysis, aiding in the processing and comparative analysis of the different images from different regions captured at different moments of the evolution of the disease/treatment. The preprocessed image (PI) file generated in the pre-processing unit (30) is transferred via a communication protocol to a remote processing unit (40), using encryption.

The remote processing unit (40) is stored on a cloud server, having a computer program using artificial intelligence techniques and algorithms, which performs the final processing of the images (FPI).

The method for identifying, measuring and evaluating the therapeutic response of the extent and distribution of the tumor burden in the peritoneal space, described in the present invention and using the detailed system, comprises an initial step of referencing an area of interest (100a) in the peritoneal space (100).

In the space of referencing the peritoneal space (100), at least one marker (10) is implanted. In a preferred embodiment, for a broad analysis of the peritoneal space (100), a quadrant is preferably defined in the user's abdomen, with four regions of interest (100a) having at least one marker (10) installed in each region (100a).

In the step of acquisition of images of the area of interest (100a) referenced by at least one marker (10), the image acquisition unit (20) is positioned aligned with the marker (10). The alignment visualization is checked on the interface of the pre-processing unit (30) connected to the image acquisition unit (20).

The images obtained in the area of interest (100a) are sent to the pre-processing unit (30) where they are transformed into the processing format required by the processing unit (40).

In subsequent captures, the original image (OI) obtained at each moment is validated following the pre-processing template of the described method. At this step, the original image (OI) goes through the same pre-processing process until it is sent to the remote processing unit (40) for the final analysis of the parameters of interest in the image.

The set of pre-processed images (PI) of a given area of interest (100a) is associated with the respective marker (10), constituting an identified file that is transferred to a remote processing unit (40) using communication protocols.

The image files consist of personal and confidential information, therefore the data are represented in the medical information standard of the DICOM system, but not limited to this format.

Illustratively, the encrypted digital file follows a pattern, for example, PACID23_0001_T01_CLIP1_R01, where the first alphanumeric sequence (PACID23) is the user identifier, the next numeric sequence (0001_T01) corresponds to the application, the second alphanumeric sequence (CLIP1) comprises the identification of the marker (10) and the third alphanumeric sequence (R01) comprises the quadrant of application of the marker (10) in the peritoneal area.

In the processing unit (40), through a computer program that executes artificial intelligence algorithms, the pre-processed images (PPI) undergo several processing steps.

In a first processing step, the algorithm is executed to obtain the initial zero image layer. This identifies the nodules, areas of interest and the marker (10). Based on the marker (10), a zero layer standard comparison mask is defined containing all oncological risk pixels (GPOR) of interest in the preprocessed image (PPI). In this first step it is possible to obtain the total number of recognizable nodules in the area of interest (100a).

In the sequential algorithm of the first processing step, the preprocessed image (PPI) is converted to black and white and contrast enhancement is performed. Algorithms such as Gray Level Transformation and/or Histogram Equalization, among others, are preferably used.

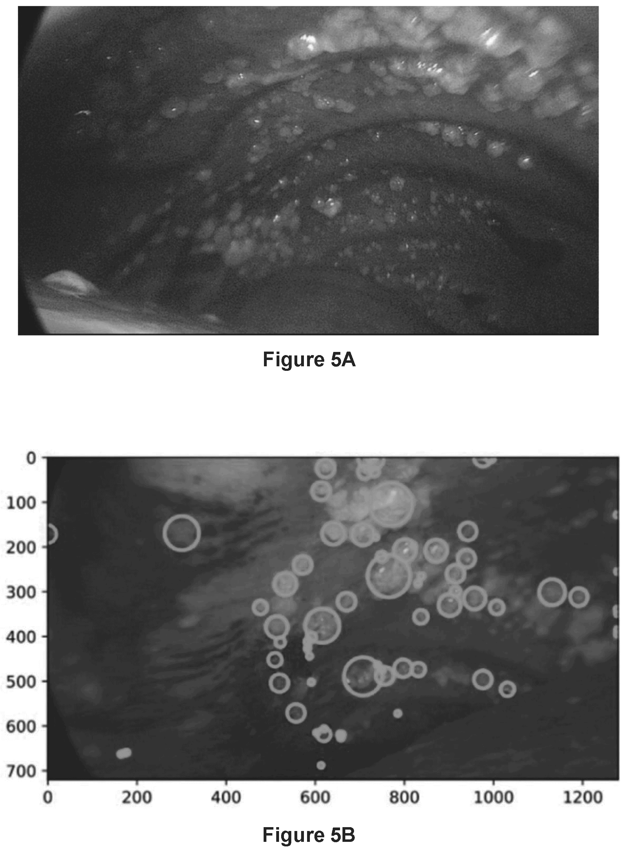

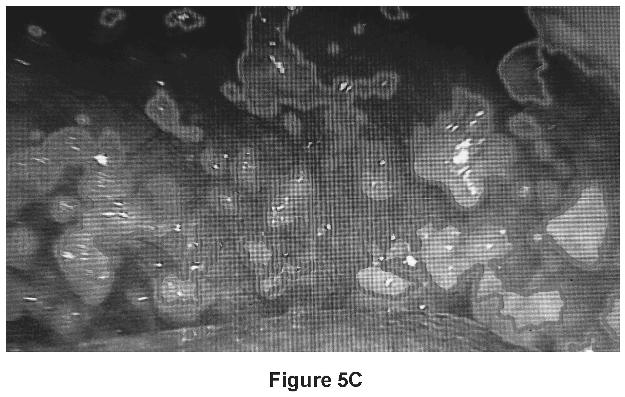

Subsequentially, in the processing algorithm, digital filters are applied in order to create edge detection by highlighting image features. Preferably, the Sobel, Laplacian or Gaussian algorithms are applied. For the process of digital identification of carcinomatosis, the calculation of the Difference of Gaussians (DoG) between the different pixels of the zero-layer images is performed, obtaining images with approximately circular regions, as shown in Figure 5A. From this analysis, a Blob (Binary Large Object) is determined. Once the blobs have been characterized and identified, libraries with the function of plotting circular regions on the image are used, as shown in Figure 5B.

Subsequentially, in the processing algorithm, digital color filters are applied to remove or enhance certain colors in the image. Preferably, the Median Filter and Mean Filter algorithms are used.

In a second processing step, layer zero is unfolded into at least one sublayer X, where the processing of pixels obtained by successive scans is carried out in order to obtain the edges of the segment. The number of sublayers X can be determined based on the number of acquired images of the area of interest (100a). At this step, the computer program recognizes the nodules, the areas of interest (100a) and the marker (10) in the sublayers X. In this second processing step, through the digital reference of the marker (10), the distance and position of the most central pixel (oncological risk pixel (ORP)) of each area of interest (100a) or nodule of interest is determined, using the recognized fixed measurement patterns on the top surface of the marker (10) defining the Peritoneal Digital Location of each of the elements of interest contained in the image.

The various sublayers X processed in the first and second steps are stored in the data ecosystem, and called, in the context of the present invention, final processed images (FPI). The final processed images (FPI) are compared to identify any changes in the image pattern.

The sublayers X are superimposed to determine the presence or absence of previous markings. Each central pixel of each image layer is compared according to its Peritoneal Digital Location, identifying changes in the recognition pattern and in the quantities analyzed in each of the sublayers X. The data obtained are stored in the processing unit database (40) for analysis and comparison with the data ecosystem, in order to identify trends and/or changes in the patterns in relation to cases with the same pathology, individual and treatment characteristics used.

To calculate the areas of the region of interest or the peritoneal nodule, each blob has its radius calculated based on the known measurements of the marker (10). From the known measurements of the marker (10), the measurement of the circular region of interest is performed, maintaining a proportional scale between the size of the known object and the size of the circular region of interest.

As a final processing step of the pre-processed image (PPI), the region of interest (100a) is segmented in order to combine distinct regions with similar specific characteristics. Preferably, the Watershed and Thresholding algorithms are used. In this identification and processing step, the thresholding technique is applied to the processed image (FPI) in order to identify regions of interest characterized by a certain homogeneity of color intensity, called, in the context of the present invention, the Group of Pixels with Oncological Risk - GPOR.

In order to perform segmentation, a convolutional neural network (CNN) is used. CNNs are a category of deep neural networks specially designed to process visual data such as images. A CNN essentially works by passing input data (image pixels) through nodes. At each node, data is weighted by adjustable parameters known as weights. These weights are responsible for capturing relevant information from the image during network training. Each node also employs activation functions, which determine how the weighted data are processed. These functions are crucial for defining and identifying important features in images, such as edges, textures, or specific patterns.

The first layer of a CNN is the convolution layer. In this layer, small filters (also called kernels) scan the original image, performing convolution operations. These operations capture simple patterns, such as edges and textures, in different parts of the image. Each filter generates a "feature map" that highlights the presence of these patterns in the image.

After the convolution layer, an activation function is applied, usually the ReLU (Rectified Linear Activation) function. This function introduces non-linearity into the network, allowing it to learn complex relationships between the identified patterns.

The pooling layer aims to reduce the dimensionality of the image, while preserving the most relevant information. A common method is Max pooling, where the image is divided into regions and only the maximum value of each region is kept, thus reducing the size of the feature maps.

After the convolution and pooling layers, the resulting feature maps are flattened into a one-dimensional vector and fed into one or more fully connected (dense) layers. These layers perform matrix operations that learn more complex and abstract representations of the features. The last layer of the network is the output layer, which produces the network's final predictions depending on the task that the CNN is performing.

To obtain the number of segmented areas, the objects (blobs) that correspond (or may correspond) to the nodules or areas of interest in the image are counted. To count objects in the images, Blob Analysis methods are used to analyze consistent regions in the image, and a complement to the technique called Detected Component Analysis may be used to identify and label the present objects in the segmented image, grouping the pixels that belong to the same object into a connected component (set of connected pixels in the image that have the same characteristics).

To delimit the regions of interest, in order to identify the present area in the segmented image, supervised or unsupervised classification techniques are used. In supervised classification, a set of labeled training data is used to teach the algorithm to identify specific patterns and classify new data into predetermined categories. Unsupervised classification does not require a labeled training dataset, and automatically groups data into categories based on common features.

To analyze the characteristics of carcinomas in the segmented image, such as size, shape and texture, feature classification algorithms are used, such as the Haralick Method, which extracts texture information from the images using statistical and mathematical techniques. The extracted features are used in machine learning models constituting a set of labeled data to recognize specific patterns in images and classify new images into predetermined categories.

## Claims

1. A system for identifying, measuring and evaluating the therapeutic response of the extent and distribution of tumor burden in the peritoneal space **characterized by** comprising the following:
a) at least one marker (10) having a top surface (11) with visual elements (111) and an anchoring means (12) associated with the top surface (11).
b) an image acquisition unit (20) that captures images of the area of interest (100a) of a peritoneal space (100) based on the referencing of at least one marker (10);
c) a pre-processing unit (30) that includes a computer board that executes a computer program that converts the original image (OI) received from the image acquisition unit (20), obtaining a preprocessed image (PI);
d) a remote processing unit installed in the cloud (40) equipped with a computer program using artificial intelligence techniques and algorithms for processing the pre-processed images (PI) in the pre-processing unit (30) and storing the final processed images (FPI).

2. A system, according to claim 1, **characterized by** the fact that the marker (10) is implanted in the peritoneal cavity (100) by means of a videolaparoscopy procedure.

3. A system, according to claim 1, **characterized by** the fact that the anchoring element (12) of the marker (10) presents indentations (121).

4. A system, according to claim 1, **characterized by** the fact that the marker (10) is positioned in a cartridge (13) equipped with a plunger (131) with a drive rod (132) that moves said marker (10) in the axial plane.

5. A system, according to claim 1, **characterized by** the fact that the image acquisition unit (20) comprises a videolaparoscopy tower and a rigid scope or rigid endoscope.

6. A system, according to claim 1, **characterized by** the fact that the pre-processing unit (20) has an interface for viewing the images captured by the acquisition unit (20) and entering data.

7. A method for identifying, measuring and evaluating the therapeutic response of the extent and distribution of tumor burden in the peritoneal space **characterized by** comprising the following steps:
a) referencing of an area of interest (100a) in the peritoneal space (100) through the implantation of at least one marker (10);
b) acquisition of images of the area of interest (100a) of the peritoneal space (100) based on the implanted marker (10);
c) validation of the original image (OI) by comparing the captured image of the marker (10) with the reference image of the marker (10);
d) modification of one or more attributes of the original image (OI), obtaining a pre-processed image (PI);
e) associating the set of pre-processed images (PI) of a given area of interest (100a) with a marker (10), constituting an identified file;
f) processing of the pre-processed images (PPI) including:
f.1) obtaining the initial zero image layer with identification of the marker (10), nodules and areas of interest;
f.2) the pre-processed image (PPI) is converted to black and white and contrast enhancement is performed;
f.3) digital filters are applied to detect edges through the enhancement of image features;
f.4) the calculation of the difference of Gaussians between the different pixels of the images of layer zero is performed;
f.5) characterization and identification of blobs;
f.6) plotting of circular regions over the zero image layer;
f.7) application of digital color filters;
f.8) unfolding of layer zero into at least one sublayer X;
f.9) determining the distance and position of the most central pixel (oncological risk pixel (ORP)) of each area of interest (100a) or nodule of interest using the fixed measurement patterns recognized on the top surface (11) of the marker (10);
g) superposition of the sublayers X with the comparison of each central pixel of each image layer according to its Peritoneal Digital Location;
h) storage of the obtained data in the processing unit database (40);
i) calculation of the area of the blobs based on the known measurements of the marker (10);
j) segmentation of the region of interest (100a) to combine distinct regions with similar specific characteristics;
k) counting the blobs that correspond to nodules or areas of interest in the image;
l) analysis of the characteristics of the blobs in the segmented image using feature classification algorithms.

8. A method, according to 7, **characterized by** the fact that the peritoneal space (100) is divided into four regions of interest (100a) having at least one marker (10) installed in each region (100a).

9. A method, according to claim 7, **characterized by** the fact that the attributes of the original image (OI) to be changed comprise adjustment of the azimuth, focus, sharpness and centering of the image on the marker (10).

10. A method, according to claim 7, **characterized by** the fact that the calculation of the areas of the region of interest or peritoneal nodule is based on the measurement of the radius of each blob compared with the known measurements of the marker (10).

11. A method, according to claim 7, **characterized by** the fact that the number of sublayers X is determined based on the number of images obtained from the area of interest (100a).

12. A method, according to claim 7, **characterized by** the fact that the counting of blobs includes the identification and labeling of the blobs present in the segmented image, with grouping of the pixels that have the same characteristics.
